# EUROPEAN PATENT APPLICATION

(11) **EP 4 837 531 A1**
(43) Date of publication of application: **11.11.2026**
(21) Application number: 24915123.4
(22) Date of filing: 19.12.2024
(51) Int. Cl.: A61B 18/02

(54) **CRYOABLATION SYSTEM, CRYOABLATION CATHETER, AND CONTROL METHOD THEREFOR**

(30) Priority: 02.01.2024 CN 202410002138
(71) Applicant: Shanghai Microport Ep Medtech Co., Ltd., Shanghai 201318 (CN)
(72) Inventor: LI, Wenhua, Shanghai 201318 (CN); YAO, Sunwu, Shanghai 201318 (CN); SUN, Yiyong, Shanghai 201318 (CN); LIANG, Bo, Shanghai 201318 (CN)
(74) Representative: Murgitroyd & Company
(86) International application number: PCT/CN2024/140594
(87) International publication number: WO 2025/145908

(57) **Abstract**

The application relates to a cryoablation system, a cryoablation catheter (10), and a control method therefor. The cryoablation catheter (10) includes an elongated shaft (100), an expandable assembly (200) disposed at a distal end of the elongated shaft (100), and a temperature measuring assembly (300). The expandable assembly (200) can occlude a vein ostium (510). The elongated shaft (100) includes an outer tube (110) and a main tube (120) which define a gas flow passage (130) therebetween. The main tube (120) has a first delivery passage extending through the main tube (120) and configured to deliver a mild liquid into a vein (50). The gas flow passage (130) is in communication with the expandable assembly (200). The temperature measuring assembly (300) is disposed on the expandable assembly (200) and can measure a temperature at the vein ostium (510) to determine effectiveness of occlusion by the expandable assembly (200).

## Description

### RELATED APPLICATIONS

This application claims priority to Chinese Patent Application No. 2024100021389, filed on January 02, 2024, entitled "CRYOABLATION SYSTEM, CRYOABLATION CATHETER, AND CONTROL METHOD THEREFOR," the content of which is incorporated herein by reference in its entirety.

### TECHNICAL FIELD

The present application relates to the field of medical device technology, and in particular, to a cryoablation system, a cryoablation catheter, and a control method therefor.

### BACKGROUND

Patients with atrial fibrillation have a high risk of cerebral stroke. During atrial fibrillation, the atria beat irregularly and rapidly, losing their contractile function, and blood tends to stagnate in the atria, leading to thrombus formation. When a thrombus dislodges and travels with the arterial blood into the brain, a cerebral stroke occurs. Through an interventional catheter, energy is applied to the pulmonary veins to perform ablation to isolate the pulmonary vein potentials, thereby achieving a therapeutic effect. Cryoballoon ablation, based on anatomical considerations, utilizes the contact between the balloon and the tissue for freezing, featuring one-time and continuous characteristics. The cryoballoon achieves low-temperature ablation mainly through the Joule-Thomson effect, which refers to the temperature drop caused by expansion and endothermic cooling when a high-pressure fluid passes through a fine capillary tube to a low-pressure region.

A cryoablation apparatus is the control integration of the entire system, mainly composed of a control panel, a refrigerant storage container, a vacuum system, and associated tubing. The refrigerant under high pressure is stored in a steel cylinder that is isolated from the outside at room temperature. At the start of cryoablation, the refrigerant is pressurized and cooled by a built-in device of the cryoablation apparatus, liquefied, and then delivered through a capillary tube into the balloon interior, where the liquefied refrigerant is sprayed out through small holes on the surface of the capillary tube and rapidly vaporizes and expands, rapidly removing heat from inside the balloon, causing a substantial temperature drop in the balloon to produce a cryoablation effect.

The effectiveness of cryoballoon ablation is based on the balloon effectively occluding the pulmonary vein ostium so that the blood in the pulmonary vein is completely blocked, thereby allowing the energy of the balloon to be effectively transferred to the tissue during ablation. Currently, during the cryoablation precedures, a contrast agent is delivered from a passage inside the catheter to the balloon end to determine whether the contrast agent leaks under X-rays, thereby determining whether the balloon is effectively occluding the ostium. However, this method requires the physician and patient to be exposed to a large amount of X-rays, posing health risks. In addition, some patients are intolerant to contrast agents (for example, allergic to contrast agents or having renal insufficiency), and the imaging system can only present two-dimensional images. When a leak occurs behind the image, the physician needs to continuously adjust the orientation to identify the specific leak location, or where the leak location may be blocked, it may easily lead to misjudgment, thereby affecting the therapeutic effect.

### SUMMARY

In view of this, a cryoablation system, a cryoablation catheter, and a control method thereof are provided, which determine whether effective occlusion is achieved by delivering a mild liquid into the vein in combination with temperature measurement, reduce the usage amount of contrast agents and X-rays, and facilitate identification of the specific leakage location when leakage occurs, so as to perform effective occlusion and ensure therapeutic effect.

A cryoablation catheter includes an elongated shaft, an expandable assembly, and a temperature measuring assembly, wherein the expandable assembly is disposed at a distal end of the elongated shaft and has an expanded configuration and a contracted configuration, and the expandable assembly, when in the expanded configuration, is capable of occluding a vein ostium;
the elongated shaft includes an outer tube and a main tube, the outer tube is sleeved over the main tube and connected at a distal end thereof to the expandable assembly, the outer tube and the main tube define a gas flow passage therebetween, the gas flow passage is in communication with the expandable assembly, the main tube has a first delivery passage extending in an axial direction, the first delivery passage extends through the main tube and is configured to deliver a mild liquid into the vein; and
the temperature measuring assembly is disposed on the expandable assembly and is capable of measuring a temperature at the vein ostium to determine effectiveness of occlusion by the expandable assembly.

In an embodiment of the present application, the expandable assembly includes a first expandable element and a second expandable element, the first expandable element envelops the second expandable element, an inner cavity of the second expandable element is in communication with the gas flow passage, and the temperature measuring assembly is disposed between the first expandable element and the second expandable element and disposed on an outer wall of the second expandable element.

In an embodiment of the present application, a plurality of temperature measuring assemblies are provided, and the plurality of temperature measuring assemblies are disposed on the expandable assembly;
a spacing is provided between adjacent temperature measuring assemblies, or the plurality of temperature measuring assemblies are distributed all over the expandable assembly.

In an embodiment of the present application, the temperature measuring assembly includes a temperature measuring element and a fixing component, the fixing component fixes the temperature measuring element to the expandable assembly, and the temperature measuring element and the fixing component are stretched along with expansion of the expandable assembly.

In an embodiment of the present application, the temperature measuring element includes a linear element and a temperature measuring point, the linear element is disposed on the fixing component, and the temperature measuring point is disposed at a distal end or an intermediate region of the linear element.

In an embodiment of the present application, the cryoablation catheter further includes a marker point, the marker point is disposed on the fixing component, and the marker point is configured to mark a relative position of the temperature measuring element with respect to the vein ostium.

In an embodiment of the present application, each of the fixing components has at least one marker point, and the marker points on the respective fixing components are different from one another.

In an embodiment of the present application, at least part of the fixing components have at least one marker point, and among the fixing components having the marker points, the marker points on the respective fixing components are different from one another.

In an embodiment of the present application, the fixing components having the marker points are adjacent to each other, and at least one fixing component having marker points is disposed between adjacent fixing components having no marker points, and at least one fixing component having no marker points is disposed between adjacent fixing components having the marker points.

In an embodiment of the present application, among the fixing components having the marker points, at least one of a number, shape, or length of the marker points is different.

A control method for a cryoablation catheter can be applied to the cryoablation catheter of any one of the above technical features, the control method includes the following steps:
inserting a distal end of the cryoablation catheter into a vein ostium;
delivering a gas to an expandable assembly to expand the expandable assembly so as to occlude the vein ostium;
injecting a mild liquid into the vein to mix with blood in the vein; and
acquiring a temperature at the vein ostium by a temperature measuring assembly and feeding back the temperature to a controller of a refrigeration device, and determining whether the expandable assembly effectively occludes the vein ostium by the controller based on a rate of temperature recovery at the vein ostium.

In an embodiment of the present application, determining whether the expandable assembly effectively occludes the vein ostium by the controller based on a temperature change at the vein ostium includes the following steps:
injecting the mild liquid into the vein, and acquiring a first temperature at the vein ostium by the temperature measuring assembly after mixing of the mild liquid with the blood ; and
after a preset time period, acquiring a second temperature at the vein ostium by the temperature measuring assembly and feeding back the second temperature to the controller, wherein the second temperature is greater than the first temperature, and determining whether the expandable assembly effectively occludes the vein ostium by the controller based on a rate of recovery from the first temperature to the second temperature.

In an embodiment of the present application, determining whether the expandable assembly effectively occludes the vein ostium by the controller based on the rate of recovery to the second temperature further includes the following steps:
the controller determining that the expandable assembly effectively occludes the vein ostium when it is determined by the controller that the second temperature is recovered at a first recovery rate;
the controller determining that the expandable assembly does not effectively occlude the vein ostium when it is determined by the controller that the second temperature is recovered at a second recovery rate;
wherein the first recovery rate is less than the second recovery rate.

In an embodiment of the present application, a plurality of temperature measuring assemblies are provided, and the plurality of temperature measuring assemblies are disposed on the expandable assembly and distributed along a circumferential direction so as to be in contact with an inner wall of the vein ostium, and the control method further includes the following steps:
injecting the mild liquid, and acquiring, by the temperature measuring assemblies, temperatures at positions of the vein ostium corresponding to the temperature measuring assemblies,
determining that the expandable assembly effectively occludes the vein ostium, when the temperatures acquired by each of the temperature measuring assemblies are recovered stably;
or when the temperatures acquired by at least one of the temperature measuring assemblies are recovered rapidly, determing that the expandable assembly has a leakage at a location corresponding to that temperature measuring assembly;
rotating the cryoablation catheter until the temperatures acquired by each of the temperature measuring assemblies are recovered stably.

A cryoablation system includes a refrigeration device, a display control device, and the cryoablation catheter of any one of the above technical features, wherein the display control device is connected to the refrigeration device, and a control handle of the cryoablation catheter has a proximal end connected to the refrigeration device and a distal end inserted into a vein ostium for occluding the vein ostium.

After adopting the above technical solutions, the present application has at least the following technical effects:
In the cryoablation system, the cryoablation catheter, and the control method therefor of the present application, a distal end of the cryoablation catheter is delivered to a pulmonary vein ostium, then a gas is delivered through the gas flow passage, so that the expandable assembly is placed in the expanded configuration and occludes the pulmonary vein ostium; at this time, a temperature of blood at the pulmonary vein ostium can be acquired by the temperature measuring assembly and fed the temperature back to the controller of the refrigeration device; then, a mild liquid is delivered into the vein through the first delivery passage, and after the mild liquid and the blood are mixed, a temperature at the pulmonary vein ostium can be measured by the temperature measuring assembly, so as to determine whether the expandable assembly effectively occludes the pulmonary vein ostium when in the expanded configuration.

The cryoablation catheter delivers the gas through the gas flow passage of the main tube to achieve expansion of the expandable assembly, so that the expandable assembly is placed in the expanded configuration and occludes the vein ostium. Moreover, before ablation, the cryoablation catheter delivers a harmless mild liquid into the vein through the first delivery passage, and the temperature measuring assembly on the expandable assembly can be used to measure temperature changes to determine the occlusion state at this time, so as to determine whether there is a leakage between the expandable assembly and the vein ostium. The usage amount of contrast agent and X-rays can also be reduced to avoid harm to the health of the physician and the patient. Moreover, the specific location of the leakage can be identified through the temperature measuring assembly, thereby helping the operator to quickly rotate and/or move the catheter, so as to adjust the direction and position of the expandable assembly for effective occlusion, facilitating subsequent ablation treatment and ensuring therapeutic effect.

### BRIEF DESCRIPTION OF THE DRAWINGS

In order to more clearly illustrate the technical solutions in the specific embodiments of the present application or in the prior art, the drawings required for describing the specific embodiments or the prior art will be briefly introduced below. Obviously, the drawings in the following description are some embodiments of the present application, and for those of ordinary skill in the art, other drawings can also be obtained based on these drawings without creative effort.
FIG. 1 is a schematic diagram of a cryoablation catheter according to an embodiment of the present application applied to a cryoablation system.
FIG. 2 is a schematic diagram of the cryoablation catheter shown in FIG. 1.
FIG. 3 is a schematic diagram of a distal end of the cryoablation catheter shown in FIG. 2 effectively occluding a vein ostium.
FIG. 4 is a schematic diagram of a distal end of the cryoablation catheter shown in FIG. 2 not effectively occluding the vein ostium.
FIG. 5 is a schematic diagram of an expandable assembly of the cryoablation catheter shown in FIG. 2 in an expanded configuration.
FIG. 6 is a top view of a distal end of the cryoablation catheter shown in FIG. 5 according to an embodiment.
FIG. 7 is a top view of a distal end of the cryoablation catheter shown in FIG. 5 according to another embodiment.
FIG. 8 is a temperature recovery curve of temperature measured by a temperature measuring assembly after effective occlusion by the cryoablation catheter shown in FIG. 2.
FIG. 9 is a temperature recovery curve of temperature measured by the temperature measuring assembly after ineffective occlusion by the cryoablation catheter shown in FIG. 2.

10, cryoablation catheter; 100, elongated shaft; 110, outer tube; 120, main tube; 121, helical opening; 130, gas flow passage; 140, flexible tube; 150, temperature sensor; 160, radiopaque member; 200, expandable assembly; 210, first expandable element; 220, second expandable element; 300, temperature measuring assembly; 310, fixing component; 320, temperature measuring element; 321, temperature measuring point; 322, linear element; 330, marker point; MT1, first temperature measuring member; MT2, second temperature measuring member; MT3, third temperature measuring member; MT4, fourth temperature measuring member; MT5, fifth temperature measuring member; MT6, sixth temperature measuring member; 400, control handle; 410, electrical interface; 420, first interface; 430, second interface; 50, vein; 510, vein ostium; 60, refrigeration device; 70, display control device.

### DETAILED DESCRIPTION

The embodiments of the present application are described in detail below with reference to the accompanying drawings in order to make the above objectives, features and advantages of the present application more apparent and understandable. Many specific details are disclosed in the following description to facilitate a comprehensive understanding of the present application. However, the present application can be implemented in many other ways different from those described herein, and those skilled in the art can make similar improvements without departing from the concept of the present application. Therefore, the present application is not limited to the specific embodiments disclosed below.

In the description of the present application, it should be understood that the terms "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", etc., indicate the orientation or position relationships based on the orientation or position relationships shown in the accompanying drawings, and are merely intended to facilitate the description of the present application and simplify the description, rather than indicating or implying that the indicated device or element must have a particular orientation or be constructed and operated in a particular orientation, and therefore are not to be interpreted as limitation on the present application.

In addition, the terms "first" and "second" are used for descriptive purposes only and cannot be interpreted as indicating or implying relative importance or implicitly specifying the number of indicated technical features. Thus, the features defined as "first" and "second" may explicitly or implicitly include at least one of these features. In the description of the present application, "plurality" means at least two, such as two, three, etc., unless otherwise clearly and specifically defined.

In the present application, unless otherwise clearly specified and limited, the terms "mount", "connection", "communication", "fix", etc., should be understood in a broad sense, for example, may be a fixed connection or a detachable connection, or an integrated connection; or may be a mechanical connection or an electrical connection; or may be a direct connection or an indirect connection through an intermediate medium; or may be an internal communication between two elements or an interaction relationship between two elements, unless otherwise clearly defined. Those of ordinary skill in the art can understand the specific meanings of the above terms in the present application according to specific situations.

In the present application, unless otherwise clearly specified and limited, a first feature being "above" or "under" a second feature may mean that the first and second features are in direct contact, or the first and second features are in indirect contact through an intermediate medium. Moreover, the first feature being "on top of", "above" and "over" the second feature may mean that the first feature is directly above or obliquely above the second feature, or simply means that the horizontal height of the first feature is greater than that of the second feature. The first feature being "under", "beneath" and "below" the second feature may mean that the first feature is directly below or obliquely below the second feature, or simply means that the horizontal height of the first feature is less than that of the second feature.

It should be noted that when an element is referred to as being "fixed on" or "disposed on" another element, the element may be directly on the other element, or there may be an intermediate element therebetween. When the element is referred to as being "connected to" another element, the element may be directly connected to the other element, or intermediate elements may also be provided. The terms "vertical", "horizontal", "upper", "lower", "left", "right" and similar expressions used herein are for the purpose of illustration only and are not meant to be the only implementation.

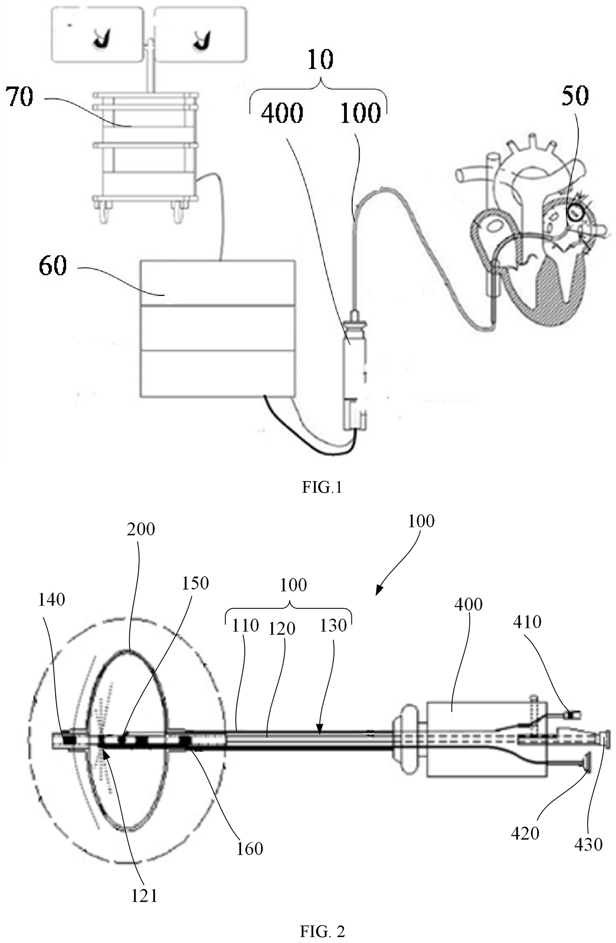

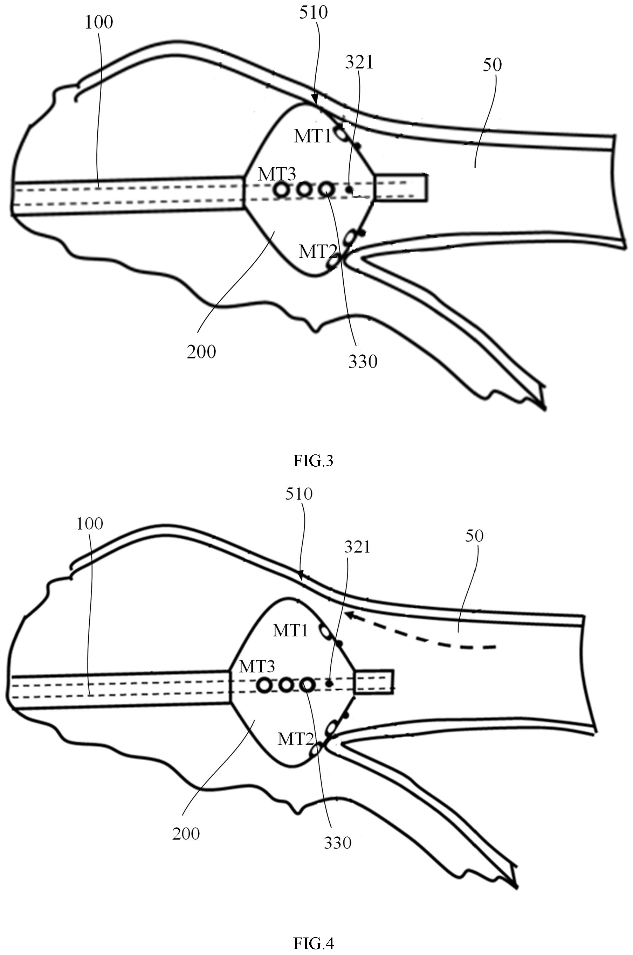

Referring to FIG. 1 to FIG. 4, the present application provides a cryoablation catheter 10. FIG. 1 is a schematic diagram of a cryoablation catheter 10 according to an embodiment of the present application applied to a cryoablation system. FIG. 2 is a schematic diagram of the cryoablation catheter 10 shown in FIG. 1. FIG. 3 is a schematic diagram of a distal end of the cryoablation catheter 10 shown in FIG. 2 effectively occluding a vein ostium 510. FIG. 4 is a schematic diagram of a distal end of the cryoablation catheter 10 shown in FIG. 2 not effectively occluding the vein ostium 510. The cryoablation catheter 10 is applied in a cryoablation system and is connected to the cryoablation system. A distal end of the cryoablation catheter 10 is capable of extending into a blood vessel and moving in the blood vessel until reaching the vein ostium 510. The cryoablation catheter 10 is capable of occluding the vein ostium 510 and performing cryoablation on a lesion site near the vein ostium 510, so as to achieve a therapeutic purpose. In the present application, the vein ostium 510 is an end of the vein 50 from which blood flows out, and the vein 50 herein refers to a pulmonary vein. Certainly, in other embodiments of the present application, the cryoablation catheter 10 may also occlude other veins or other blood vessels to perform ablation treatment on nearby lesion sites. The following description only takes the cryoablation catheter 10 occluding the vein ostium 510 as an example for illustration.

It is understandable that, currently, during the cryoablation procedure, a contrast agent is delivered through a passage inside the catheter to the balloon end to determine whether the contrast agent leaks under X-rays, thereby determining whether the balloon is effectively occluding the ostium. However, this method requires the physician and patient to be exposed to a large amount of X-rays, posing health risks. In addition, some patients are intolerant to contrast agents, and the leak location may be easily misjudged, thereby adversely affecting the therapeutic effect. Acoordingly, the present application provides a novel cryoablation catheter 10, which determines whether effective occlusion is achieved by delivering a mild liquid into the vein 50 in combination with temperature measurement, so as to avoid leakage and facilitate subsequent ablation treatment, reducing the usage amount of contrast agent and X-rays, thereby avoiding harm to the health of the physician and the patient. The specific location of the leakage can be identified readily when the leakage occurs, so that an effective occlusion can be performed to ensure therapeutic effect. The specific structure of the cryoablation catheter 10 according to an embodiment is described below.

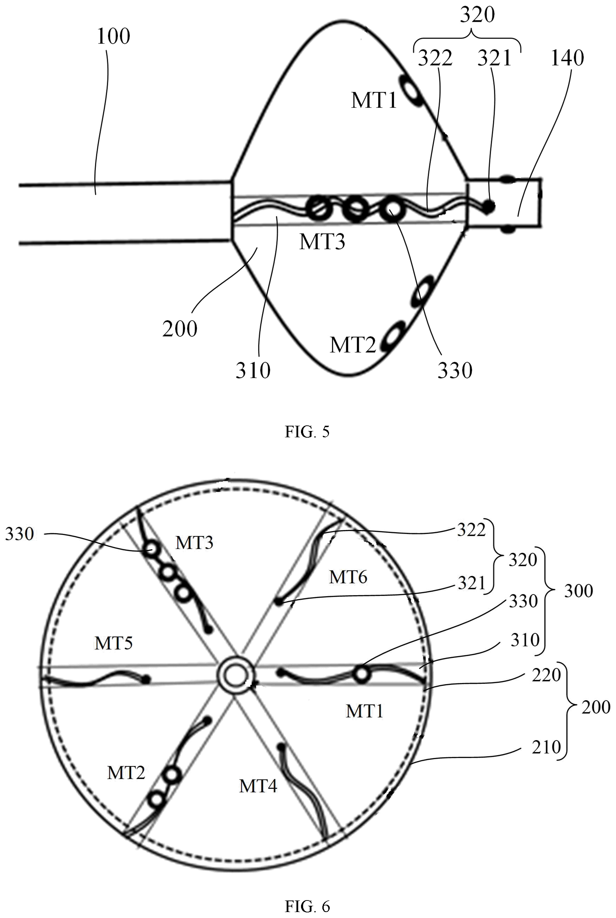

Referring to FIG. 1 to FIG. 5, in an embodiment, the cryoablation catheter 10 includes an elongated shaft 100, an expandable assembly 200, and a temperature measuring assembly 300. The expandable assembly 200 is disposed at a distal end of the elongated shaft 100 and has an expanded configuration and a contracted configuration. The expandable assembly 200, when in the expanded configuration, is capable of occluding a vein ostium 510. The elongated shaft 100 includes an outer tube 110 and a main tube 120. The outer tube 110 is sleeved over the main tube 120 and connected at a distal end thereof to the expandable assembly 200. The outer tube 110 and the main tube 120 define a gas flow passage 130 therebetween. The main tube 120 has a first delivery passage extending in an axial direction thereof. The gas flow passage 130 is in communication with the expandable assembly 200. The first delivery passage extends through the main tube 120 and is configured to deliver a mild liquid into the vein 50. The temperature measuring assembly 300 is disposed on the expandable assembly 200 and is capable of measuring a temperature at the vein ostium 510 to determine effectiveness of occlusion by the expandable assembly 200. FIG. 5 is a schematic diagram of the expandable assembly 200 of the cryoablation catheter 10 shown in FIG. 2 in the expanded configuration.

The elongated shaft 100 extends along a length direction thereof and has a proximal end and a distal end disposed opposite to each other. The proximal end refers to an end of the elongated shaft 100 close to the operator, and the distal end refers to an end of the elongated shaft 100 away from the operator and close to the patient. The axial direction and the length direction refer to a direction of a line connecting the proximal end and the distal end. It should be noted that the proximal end and the distal end of the elongated shaft 100 are also applicable to other components, which will not be described again hereinafter. The expandable assembly 200 is disposed at the distal end of the elongated shaft 100. The distal end of the elongated shaft 100 is capable of extending into the blood vessel and driving the expandable assembly 200 to move in the blood vessel, so as to deliver the expandable assembly 200 to the vein ostium 510.

As shown in FIG. 2, the outer tube 110 is sleeved over the main tube 120 to form a main structure of the elongated shaft 100. A spacing exists between an inner wall of the outer tube 110 and an outer wall of the main tube 120, and the inner wall and the outer wall define the gas flow passage 130 therebetween. The gas flow passage 130 is capable of allowing a gas and a gaseous refrigerant to flow therethrough. Distal ends of the outer tube 110 and the main tube 120 are connected to the expandable assembly 200, forming an annular gas flow passage 130 with an open proximal end and a closed distal end. An inner cavity of the expandable assembly 200 is in communication with the gas flow passage 130. The expandable assembly 200 has an expanded configuration and a contracted configuration. When the expandable assembly 200 is in the contracted configuration, delivery of the expandable assembly 200 in the blood vessel by the elongated shaft 100 can be facilitated. When the gas is delivered into the expandable assembly 200 through the gas flow passage 130 after the elongated shaft 100 delivers the expandable assembly 200 to the vein ostium 510, the expandable assembly 200 is switched from the contracted configuration to the expanded configuration. In this case, the expandable assembly 200 is capable of occluding the vein ostium 510, preventing blood from flowing out of the vein 50 through the vein ostium 510, so as to facilitate subsequent cryoablation operation.

The main tube 120 is hollow, and has a first delivery passage and a second delivery passage which are hollow and independent of each other. The first delivery passage and the second delivery passage extend along the axial direction. The first delivery passage extends through the main tube 120, that is, the first delivery passage has a proximal end extending to the proximal end of the main tube 120, and a distal end thereof extending to the distal end of the main tube 120, with both ends of the first delivery passage being open. After the expandable assembly 200 occludes the vein ostium 510, the distal end of the main tube 120 is located in the vein 50. Then, the mild liquid enters from the proximal end of the first delivery passage, flows through the first delivery passage, and is delivered into the vein 50 through the distal end of the first delivery passage. The mild liquid is mixed with blood in the vein 50 to determine whether the expandable assembly 200 effectively occludes the vein ostium 510. When the expandable assembly 200 effectively occludes the vein ostium 510, the expandable assembly 200 is in sealing contact with the vein ostium 510, preventing blood from leaking from the vein ostium 510. When the expandable assembly 200 does not effectively occlude the vein ostium 510, a gap exists between the expandable assembly 200 and the vein ostium 510 in at least one position, and blood in the vein 50 will leak through the gap, affecting subsequent ablation treatment. In this case, it is necessary to move and/or rotate the cryoablation catheter 10, so as to adjust the direction and position of the expandable assembly 200 until the expandable assembly 200 effectively occludes the vein ostium 510, thereby facilitating the subsequent ablation treatment.

A distance is provided between the second delivery passage and the distal end of the main tube 120. The second delivery passage is in communication with the expandable assembly 200. The second delivery passage is in communication with the gas flow passage 130. The gas flow passage is configured to deliver gas during gas delivery, and the gas flow passage is capable of delivering gas to the expandable assembly 200. In this case, the expandable assembly 200 is capable of occluding the vein ostium 510. The second delivery passage is configured to deliver refrigerant, and the gas flow passage is also capable of outputting gaseous refrigerant after cryoablation. After the expandable assembly 200 completely occludes the vein ostium 510, the second delivery passage is capable of delivering refrigerant to the expandable assembly 200 for cryoablationoperation. Specifically, the refrigerant enters from the proximal end of the second delivery passage, and is then delivered into the inner cavity of the expandable assembly 200 to perform cryoablation operation at the vein ostium 510. After cryoablation, the refrigerant becomes gaseous and flows out through the gas flow passage 130.

Referring to FIG. 3 to FIG. 5, in the present application, the temperature measuring assembly 300 is provided on the expandable assembly 200 to detect the effectiveness of occlusion. The temperature measuring assembly 300 is electrically connected to a controller of the refrigeration device 60. After the expandable assembly 200 occludes the vein ostium 510, the temperature measuring assembly 300 is located at the vein ostium 510. The temperature measuring assembly 300 is capable of measuring a temperature of blood at the vein ostium 510 and feeding the temperature back to the controller. The controller determines effectiveness of occlusion of the vein ostium 510 by the expandable assembly 200 according to the temperature. After the expandable assembly 200 effectively occludes the vein ostium 510, ablation treatment can be performed on the lesion site around the vein ostium 510. When the expandable assembly 200 does not effectively occlude the vein ostium 510, blood will flow out of the vein 50 through the vein ostium 510. In this case, it is necessary to adjust the direction and position of the expandable assembly 200 so that the expandable assembly 200 occludes the vein ostium 510.

It is understandable that the logic for determining whether effective occlusion is achieved is: starting once the temperature measured by each temperature measuring assembly 300 on the surface of the expandable assembly 200 drops to a lowest temperature, the rate or amplitude of temperature recovery within a certain period of time is calculated to determine the occlusion state of the vein 50 by the expandable assembly 200. When the temperature is recovered stably, at a slow recovery rate and with a small amplitude, the expandable assembly 200 effectively occludes the vein ostium 510. When the temperature is recovered rapidly, at a fast recovery rate and with a large amplitude, the expandable assembly 200 does not effectively occlude the vein ostium 510. Hereinafter, only the recovery rate is used as an example for illustration. When the recovery rate is fast, the temperature is recovered rapidly; when the recovery rate is slow, the temperature is recovered stably.

When the expandable assembly 200 completely occludes the vein ostium 510, blood in the vein 50 cannot flow into the atrium. After a certain amount of mild liquid is injected into the main tube 120 through the first delivery passage, the mild liquid flows through the distal end of the first delivery passage into the blood in the vein 50. In this case, the mild liquid cannot flow into the atrium. As the mild liquid and the blood gradually mix, the temperature in the vein 50 gradually decreases from the initial temperature of the blood. The blood temperature after decreasing is measured by the temperature measuring assembly 300. After it is measured by the temperature measuring assembly 300 that the blood drops to the lowest temperature, the temperature of the blood will be maintained for a period of time or slowly recovered under the influence of body temperature, with a relatively small recovery amplitude within a certain period of time. That is, after the expandable assembly 200 completely occludes the vein ostium 510, stable recovery would be detected by the temperature measuring assembly 300 after detecting the lowest temperature, indicating that the expandable assembly 200 effectively occludes the vein ostium 510, as shown in FIG. 3 and FIG. 5.

When the expandable assembly 200 does not completely occlude the vein ostium 510, a certain gap exists between the vein ostium 510 and the expandable assembly 200, and blood in the vein 50 flows to the atrium through the gap. After the expandable assembly 200 is inflated to the expandable configuration, a certain amount of mild liquid is injected into the main tube 120 through the first delivery passage. The mild liquid flows through the distal end of the first delivery passage into the blood in the vein 50. After the mild liquid is gradually mixed with the blood, part of the mixture flows into the atrium through the gap between the expandable assembly 200 and the vein ostium 510. Under the flushing of the blood flow, the temperature at the vein ostium 510 would be recovered at a relatively fast rate after dropping to the lowest point, with a relatively large recovery amplitude within a certain period of time. That is, when the expandable assembly 200 does not completely occlude the vein ostium 510, rapid recovery would be detected by the temperature measuring assembly 300 at the gap after detecting the temperature dropping to the lowest temperature, indicating that the expandable assembly 200 does not effectively occlude the vein ostium 510. In this case, the location of the gap can be determined according to the difference in temperature recovery rates of the temperature measuring assemblies 300, and the operator can then adjust the direction and position of the expandable assembly 200 to ensure complete occlusion of the vein ostium 510.

The cryoablation catheter 10 allows the use of the mild liquid, instead of the contrast agent, to determine the occlusion state of the vein ostium 510 by detecting temperature changes on the surface of the expandable assembly 200 through the temperature measuring assembly 300. The final occlusion state is displayed on a display screen of the display control device 70 through the controller in an intuitive manner. When it is determined by the controller that the temperature detected by the temperature measuring assembly 300 is recovered stably, it indicates that the expandable assembly 200 can effectively occlude the vein ostium 510. When it is determined by the controller that the temperature detected by the temperature measuring assembly 300 is recovered rapidly, it indicates that the expandable assembly 200 does not effectively occlude the vein ostium 510. In this case, there is a leakage in at least one location of the expandable assembly 200, and the orientation of the cryoablation catheter 10 needs to be adjusted until the expandable assembly 200 effectively occludes the vein ostium 510. Moreover, the mild liquid does not cause damage to the blood vessels, and can effectively avoid the situation where patients are intolerant to contrast agents, reduce X-ray irradiation, and avoid damage to the patient and the operator, as shown in FIG. 4 and FIG. 5.

Referring to FIG. 1 to FIG. 5, when the cryoablation catheter 10 is used, the distal end of the cryoablation catheter 10 is inserted into the blood vessel, and the elongated shaft 100 is advanced so that the expandable assembly 200 is delivered to the vein ostium 510. Then, the gas is delivered from the gas flow passage 130 into the expandable assembly 200, so that the expandable assembly 200 is switched from the contracted configuration to the expanded configuration. In this case, the expandable assembly 200 is capable of occluding the vein ostium 510. After the expandable assembly 200 occludes the vein ostium 510, it is necessary to determine the effectiveness of occlusion of the vein ostium 510. The mild liquid is delivered from the first delivery passage into the vein 50. The temperature of blood at the vein ostium 510 is measured by the temperature measuring assembly 300 and fed the temperatue back to the controller to determine whether the expandable assembly 200 effectively occludes the vein ostium 510. After complete occlusion, the refrigerant is delivered to the expandable assembly 200 through the second delivery passage, so that cryoablation can be performed with the cryoablation catheter 10 on the lesion site near the vein ostium 510, so as to achieve the therapeutic purpose.

In the cryoablation catheter 10 of the above embodiment, the gas is delivered through the gas flow passage 130 of the main tube 120 to achieve expansion of the expandable assembly 200, so that the expandable assembly 200 is placed in the expanded configuration and occludes the vein ostium 510. During cryoablation, the cryoablation operation can be achieved through the cooperation of the gas flow passage, the second delivery passage, and the expandable assembly 200. Moreover, before ablation, the cryoablation catheter 10 delivers a harmless mild liquid into the vein 50 through the first delivery passage, and the temperature measuring assembly 300 on the expandable assembly 200 can be used to measure temperature changes to determine the occlusion state at this time, so as to determine whether there is a leakage between the expandable assembly 200 and the vein ostium 510. The usage amount of contrast agents and X-rays can also be reduced to avoid harm to the health of the physician and the patient. Moreover, the specific location of the leakage can be identified through the temperature measuring assembly 300, thereby helping the operator to quickly rotate and/or move the catheter, so as to adjust the direction and position of the expandable assembly 200 for effective occlusion, facilitating subsequent ablation treatment and ensuring therapeutic effect.

Optionally, the mild liquid is physiological saline, a balanced salt solution, or other types of mild liquid. In these embodiments, the mild liquid is harmless physiological saline at room temperature. Using physiological saline instead of the contrast agent can, in cooperation with the temperature measuring assembly 300, determine whether the expandable assembly 200 effectively occludes the vein ostium 510, while also avoiding the intolerance caused by the use of contrast agents, and reducing X-ray irradiation. Certainly, in other embodiments of the present application, the mild liquid may also be the balanced salt solution or other types of mild liquid, as long as it can, in cooperation with the temperature measuring assembly 300, determine whether effective occlusion is achieved.

Referring to FIG. 2, optionally, the main tube 120 has a helical opening 121. The helical opening 121 is in communication with the second delivery passage and the gas flow passage 130. In this way, the refrigerant in the second delivery passage can enter the expandable assembly 200 in a helical direction through the helical opening 121, so that the refrigerant can be delivered to the expandable assembly 200 from various directions to meet cryoablation requirements. Optionally, the cryoablation catheter 10 further includes a control handle 400. The control handle 400 is disposed at the proximal end of the elongated shaft 100, and the control handle 400 is configured to control the movement of the elongated shaft 100 so as to deliver the cryoablation catheter 10 to a desired position. Optionally, the cryoablation catheter 10 further includes a flexible tube 140. The flexible tube 140 is disposed at the distal end of the elongated shaft 100 and located at the distal end of the expandable assembly 200. The flexible tube 140 can provide flexible guidance to avoid scratching the blood vessel. Optionally, the control handle 400 has an electrical interface 410. The electrical interface 410 is disposed at the proximal end of the control handle 400 and is electrically connected to the temperature measuring assembly 300 through a lead wire. The lead wire is arranged in an insulated manner in the gas flow passage 130, or is routed through an insulating tube to form a wire routing passage, or is routed through the outer tube 110 or the main tube 120.

Referring to FIG. 2, optionally, the control handle 400 further includes a first interface 420. The first interface 420 is disposed at the proximal end of the control handle 400 and is in communication with the proximal end of the second delivery passage for delivering refrigerant to the second delivery passage. The first interface 420 is in communication with a storage tank of the refrigeration device 60 that stores the refrigerant. Optionally, the control handle 400 further includes a second interface 430. The second interface 430 is disposed at the proximal end of the control handle 400 and is in communication with the proximal end of the first delivery passage for delivering the mild liquid to the first delivery passage. Certainly, the first delivery passage can also allow a guiding instrument to pass therethrough. Optionally, the elongated shaft 100 further includes a temperature sensor 150. The temperature sensor 150 is disposed on the outer wall of the main tube 120 and located in the expandable assembly 200. The temperature sensor 150 is configured to measure the temperature during cryoablation. Optionally, the cryoablation catheter 10 further includes a radiopaque member 160. The radiopaque member 160 is disposed on the outer tube 110 for positioning the position of the cryoablation catheter 10 in the blood vessel, ensuring that the distal end of the cryoablation catheter 10 can be accurately moved to the vein ostium 510.

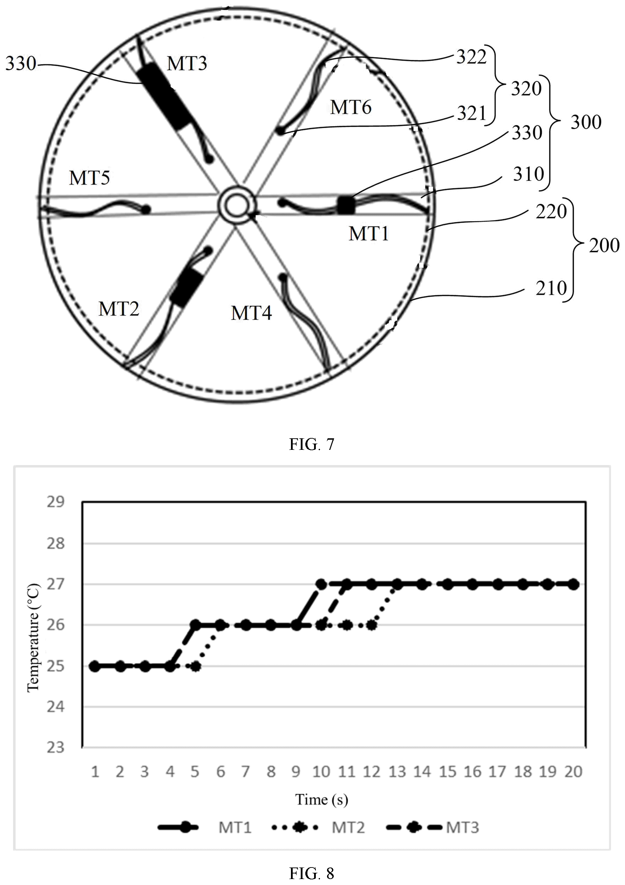

Referring to FIG. 5 to FIG. 7, the expandable assembly 200 includes a first expandable element 210 and a second expandable element 220. The first expandable element 210 envelops the second expandable element 220. An inner cavity of the second expandable element 220 is in communication with the gas flow passage 130. The temperature measuring assembly 300 is disposed between the first expandable element 210 and the second expandable element 220 and disposed on an outer wall of the second expandable element 220. FIG. 6 is a top view of the distal end of the cryoablation catheter 10 shown in FIG. 5 according to an embodiment. FIG. 7 is a top view of the distal end of the cryoablation catheter 10 shown in FIG. 5 according to another embodiment.

The first expandable element 210 and the second expandable element 220 each have a contracted configuration and an expanded configuration, and are disposed in a sleeved manner. The first expandable element 210 is sleeved outside the second expandable element 220, and an inner wall of the first expandable element 210 is in fitted connection with an outer wall of the second expandable element 220. Thus, a double-layer expandable assembly 200 is formed. After the gas enters the gas flow passage 130, it can enter the inner cavity of the second expandable element 220, and control the second expandable element 220 to gradually switch from the contracted configuration to the expanded configuration. During the expansion of the second expandable element 220, it can drive the first expandable element 210 to expand, so that the expandable assembly 200 as a whole is in the expanded configuration. At this time, the first expandable element 210 can come into contact with the vein ostium 510 to achieve occlusion of the vein ostium 510.

After the second delivery passage delivers the refrigerant into the inner side of the second expandable element 220, the refrigerant can perform cryoablation on the lesion site surrounding the vein ostium 510, and the gaseous refrigerant after cryoablation flows out through the gas flow passage 130. The temperature measuring assembly 300 is disposed on the outer wall of the second expandable element 220 and is in contact with the inner wall of the first expandable element 210. The temperature at the vein ostium 510 can be measured by temperature measuring assembly 300 in real time. Meanwhile, the first expandable element 210 can also provide protection, protecting the temperature measuring assembly 300 and the second expandable element 220. Optionally, the first expandable element 210 and the second expandable element 220 are both balloons or other components capable of expansion and contraction.

Referring to FIG. 5 to FIG. 7, in an embodiment, a plurality of temperature measuring assemblies 300 are provided, and the plurality of temperature measuring assemblies 300 are disposed on the expandable assembly 200. The plurality of temperature measuring assemblies 300 are disposed on the outer wall of the second expandable element 220, and the temperature measuring assemblies 300 extend from the proximal end to the distal end. A spacing is provided between adjacent temperature measuring assemblies 300. That is, temperatures at different positions can be measured by the plurality of temperature measuring assemblies 300. When the expandable assembly 200 is in contact with the vein ostium 510, the plurality of temperature measuring assemblies 300 are in contact with the vein ostium 510 in the circumferential direction of the vein ostium 510. In this way, temperatures at different positions of the vein ostium 510 can be monitored by the plurality of temperature measuring assemblies 300 so as to detect the effectiveness of occlusion at various circumferential positions of the expandable assembly 200.

After the expandable assembly 200 occludes the vein ostium 510, the plurality of temperature measuring assemblies 300 are distributed at different positions of the vein ostium 510. After the mild liquid is introduced, each temperature measuring assembly 300 can respectively acquire a temperature change at a corresponding position of the vein ostium 510. When the temperatures acquired by each of the temperature measuring assemblies 300 are recovered stably, the controller determines that the expandable assembly 200 effectively occludes the vein ostium 510. When the temperature acquired by at least one temperature measuring assembly 300 is recovered rapidly, it indicates that a gap exists between the expandable assembly 200 and the vein ostium 510 at the position corresponding to that temperature measuring assembly 300, and effective occlusion is not achieved. In this case, it is necessary to adjust the direction and position of the expandable assembly 200 until the temperatures acquired by each of the temperature measuring assemblies 300 are recovered stably, so as to achieve effective occlusion of the vein ostium 510.

In an embodiment, a spacing is provided between adjacent temperature measuring assemblies 300, or the plurality of temperature measuring assemblies 300 are distributed all over the expandable assembly 200. The plurality of temperature measuring assemblies 300 are provided on the expandable assembly 200 with spacings therebetween, as long as the plurality of temperature measuring assemblies 300 are distributed in the circumferential direction of the expandable assembly 200 and can meet the temperature detection requirements at various circumferential positions of the expandable assembly 200. Certainly, the plurality of temperature measuring assemblies 300 may also be distributed all over the expandable assembly 200, so that the temperature detection at various circumferential positions of the expandable assembly 200 can be achieved.

Referring to FIG. 5 to FIG. 7, in an embodiment, the temperature measuring assembly 300 includes a temperature measuring element 320 and a fixing component 310. The fixing component 310 fixes the temperature measuring element 320 to the expandable assembly 200. The temperature measuring element 320 and the fixing component 310 are stretched along with expansion of the expandable assembly 200. The temperature measuring element 320 is disposed on a surface of the fixing component 310. The fixing component 310 is disposed on the second expandable element 220, thereby fixing the temperature measuring element 320 to the second expandable element 220. During expansion of the second expandable element 220, it can drive the fixing component 310 and the temperature measuring element 320 to be stretched synchronously, so that the temperature measuring element 320 can be in contact with the blood to meet temperature measurement requirements. Optionally, the fixing component 310 is in a strip shape after being stretched, and the temperature measuring element 320 is disposed on the fixing component 310. Optionally, the temperature measuring element 320 is absorbed and fixed on the outer wall of the second expandable element 220 through the fixing component 310.

Referring to FIG. 5 to FIG. 7, in an embodiment, the temperature measuring element 320 includes a linear element 322 and a temperature measuring point 321. The linear element 322 is disposed on the fixing component 310. A distal end of the linear element 322 is electrically connected to the temperature measuring point 321 for transmitting a temperature signal detected by the temperature measuring point 321. A proximal end of the linear element 322 is electrically connected to the controller through a lead wire, so as to feed back the temperature signal measured by the temperature measuring element 320 to the controller, so that the controller can determine whether the vein ostium 510 is effectively occluded. The temperature measuring point 321 is disposed at an end or an intermediate portion of the linear element 322, so as to facilitate detection of the temperature of blood. Moreover, the temperature measuring point 321 can be at any position of the linear element 322.

It should be noted that the temperature measuring point 321 can be disposed at any position of the second expandable assembly 200. Optionally, the temperature measuring point 321 is located on a distal spherical surface of the expandable assembly 200. In this way, when the expandable assembly 200 occludes the vein ostium 510, the temperature measuring point 321 is located in the vein 50 as much as possible, ensuring temperature measurement effectiveness. Optionally, the temperature measuring point 321 is disposed at a distal end or an intermediate region of the linear element 322. Optionally, the temperature measuring point 321 is a temperature measuring solder joint or a temperature measuring sensor, etc. Certainly, in other embodiments of the present application, the temperature measuring point 321 may also be disposed on the flexible tube 140.

Referring to FIG. 5 to FIG. 7, in an embodiment, the cryoablation catheter 10 further includes a marker point 330. The marker point 330 is disposed on the fixing component 310. The marker point 330 is capable of marking a relative position of the temperature measuring element 320 on the expandable assembly 200 under X-rays. The marker point 330 is capable of marking the position and direction of different temperature measuring elements 320 for identification by the operator. When a gap exists between the expandable assembly 200 and the vein ostium 510 at the position where the temperature measuring assembly 300 is located, the leakage location can be accurately determined by identifying the marker point 330, so as to facilitate adjustment of the expandable assembly 200. The marker point 330 is identified in cooperation with X-rays. Optionally, the marker point 330 is made of a material that is visible under X-rays.

In an embodiment, each of the fixing components 310 has at least one marker point 330, and the marker points 330 on the respective fixing components 310 are different from one another. That is, each fixing component 310 is provided with a different marker point 330. The position corresponding to temperature measuring assembly 300 can be identified by the corresponding marker point 330, so as to determine whether the expandable assembly 200 at that position effectively seals the vein ostium 510. Once the temperature of a certain temperature measuring assembly 300 is recovered rapidly, the controller can easily determine that effective occlusion is not achieved at the position of the temperature measuring assembly 300, and then adjust the orientation of the expandable assembly 200 until the expandable assembly 200 completely occludes the vein ostium 510.

Referring to FIG. 5 to FIG. 7, in an embodiment, at least part of the fixing components 310 have at least one marker point 330. Among the fixing components 310 having the marker points 330, the marker points 330 on the respective fixing components 310 are different from one another. That is, only part of the fixing components 310 have different marker points 330, and the remaining fixing components 310 have no marker points 330. In this case, the fixing components 310 with marker points 330 can be positioned through the marker points 330 thereon. The fixing components 310 without marker points 330 can be positioned through adjacent fixing components 310 having the marker points 330. As shown in FIG. 6 and FIG. 7, part of the fixing components 310 have the marker points 330, and part of the fixing components 310 do not have the marker points 330.

Referring to FIG. 5 to FIG. 7, in an embodiment, among the fixing components 310 having the marker points 330, at least one of the number, shape, and length of the marker points 330 on each of the fixing components 310 is different. That is, among the fixing components 310 having the marker points 330, the marker points 330 on respective fixing component 310 are different from one another, so as to distinguish the relative positions on the expandable assembly 200 corresponding to the marker points 330. Exemplarily, as shown in FIG. 6, the fixing components 310 having the marker points 330 are distinguished from one another by different numbers of the marker points 330. Exemplarily, the fixing components 310 having the marker points 330 are distinguished from one another by different lengths of the marker points 330.

Referring to FIG. 5 to FIG. 7, in an embodiment, the fixing components 310 having the marker points 330 are adjacent to each other, and at least one fixing component 310 having the marker point 330 is disposed between adjacent fixing components 310 having no marker points 330, and at least one fixing component 310 having no marker points 330 is disposed between adjacent fixing components 310 having the marker points 330. That is, at least one fixing component 310 without a marker point 330 is disposed between two adjacent fixing components 310 with marker points 330, and at least one fixing component 310 with a marker point 330 is disposed between two adjacent fixing components 310 without marker points 330.

Referring to FIG. 5 to FIG. 7, exemplarily, the fixing components 310 having the marker points 330 and the remaining fixing components 310 are arranged alternately. That is, one fixing component 310 without marker points 330 is disposed between two adjacent fixing components 310 having marker points 330, and one fixing component 310 having marker point(s) 330 is disposed between two adjacent fixing components 310 without marker points 330. In this way, the positions of each temperature measuring assembly 300 on the expandable assembly 200 can be easily determined, thereby facilitating determination of effectiveness of occlusion.

For convenience of describing the principle of effective occlusion by the plurality of temperature measuring assemblies 300, the number of temperature measuring assemblies 300 is taken as 6 for illustration herein, among which the three having marker points 330 are respectively a first temperature measuring member MT1, a second temperature measuring member MT2, and a third temperature measuring member MT3, and the three without marker points 330 are respectively a fourth temperature measuring member MT4, a fifth temperature measuring member MT5, and a sixth temperature measuring member MT6. The first temperature measuring member MT1, the second temperature measuring member MT2, and the third temperature measuring member MT3 are arranged alternately with the fourth temperature measuring member MT4, the fifth temperature measuring member MT5, and the sixth temperature measuring member MT6. The fourth temperature measuring member MT4 is disposed between the first temperature measuring member MT1 and the second temperature measuring member MT2. The fifth temperature measuring member MT5 is disposed between the second temperature measuring member MT2 and the third temperature measuring member MT3. The sixth temperature measuring member MT6 is disposed between the first temperature measuring member MT1 and the third temperature measuring member MT3.

The marker points 330 on the first temperature measuring member MT1, the second temperature measuring member MT2, and the third temperature measuring member MT3 are different. As shown in FIG. 6, the first temperature measuring member MT1 has one marker point 330, the second temperature measuring member MT2 has two marker points 330, and the third temperature measuring member MT3 has three marker points 330. As shown in FIG. 7, the marker points 330 on the first temperature measuring member MT1, the second temperature measuring member MT2, and the third temperature measuring member MT3 are in a strip shape, with lengths increasing sequentially.

When the expandable assembly 200 completely occludes the vein ostium 510, the temperatures of the first temperature measuring member MT1 to the sixth temperature measuring member MT6 are recovered stably after a preset time period from introduction of the mild liquid. In this case, the expandable assembly 200 effectively occludes the vein ostium 510. Assuming that the expandable assembly 200 does not completely occlude the vein ostium 510 at the first temperature measuring member MT1, blood will flow out from the gap between the expandable assembly 200 and the vein ostium 510 at the first temperature measuring member MT1, and the temperature measured by the first temperature measuring member MT1 is recovered rapidly. At the same time, the temperatures at the second temperature measuring member MT2 and the third temperature measuring member MT3 are recovered stably. Thus, it indicates that the expandable assembly 200 does not effectively occlude the vein ostium 510, and leakage occurs at the first temperature measuring member, while effective occlusion is achieved at the other temperature measuring positions. In this case, the orientation of the expandable assembly 200 is adjusted so that the expandable assembly 200 effectively occludes at each temperature measuring member. The principle of leakage at the second temperature measuring member MT2 and the third temperature measuring member MT3 is substantially the same and will not be repeated here.

When the expandable assembly 200 does not completely occlude the vein ostium 510 at the fourth temperature measuring member MT4, the temperature measured by the fourth temperature measuring member MT4 is recovered rapidly. Since the fourth temperature measuring member MT4 is located between the first temperature measuring member MT1 and the second temperature measuring member MT2, and the first temperature measuring member MT1 and the second temperature measuring member MT2 achieve effective occlusion, leakage occurring between the first temperature measuring member MT1 and the second temperature measuring member MT2 indicates that the fourth temperature measuring member MT4 does not effectively occlude the vein ostium 510. When effective occlusion is achieved at the other positions, the orientation of the expandable assembly 200 is adjusted so that the expandable assembly 200 effectively occludes at each temperature measuring member. The principle of leakage at the fifth temperature measuring member MT5 and the sixth temperature measuring member MT6 is substantially the same and will not be repeated here.

Referring to FIG. 3 and FIG. 8, FIG. 8 is a temperature recovery curve of temperature measured by the temperature measuring assembly 300 after effective occlusion by the cryoablation catheter 10 shown in FIG. 2. When the expandable assembly 200 completely occludes the vein ostium 510, blood in the vein 50 cannot flow into the atrium. After a certain amount of mild liquid is injected into the main tube 120 through the first delivery passage, the mild liquid flows through the distal end of the first delivery passage into the blood in the vein 50. At this time, the mild liquid cannot flow into the atrium. As the mild liquid is gradually mixed with the blood , the temperature in the vein 50 gradually decreases from the initial temperature of the blood. At this time, temperature changes are recorded by the first temperature measuring member MT1, the second temperature measuring member MT2, and the third temperature measuring member MT3, and the recorded data are calculated and determined by the controller. Finally, the occlusion state of the expandable assembly 200 is displayed on the display screen. Generally, the determination logic is: starting once the temperature measured by each temperature measuring point 321 on the surface of the balloon drops to a lowest temperature, the rate or amplitude of temperature recovery within a certain period of time is calculated to determine the occlusion state of the vein 50. In this case of complete occlusion, blood in the vein 50 cannot flow into the left atrium. Therefore, after a certain amount of physiological saline is injected, the temperature at the vein ostium 510 drops to the lowest point and will then be maintained for a period of time or slowly recovered under the influence of body temperature, with a relatively small recovery amplitude within a certain period of time. FIG. 8 is a temperature change curve of the surface temperature when the balloon is completely occluded, and the temperature at each point on the surface is recovered slowly within 20 seconds. The blood temperature after decreasing is measured by the temperature measuring assembly 300. After the blood drops to the lowest temperature, the temperature of the blood will be maintained for a period of time or slowly recovered under the influence of body temperature, with a relatively small recovery amplitude within a certain period of time. That is, after the expandable assembly 200 completely occludes the vein ostium 510, stable recovery would be detected by the temperature measuring assembly 300 after detecting the lowest temperature, indicating that the expandable assembly 200 effectively occludes the vein ostium 510, as shown in FIG. 3 and FIG. 8. After 20 seconds, the temperatures of the respective temperature measuring members are recovered slowly.

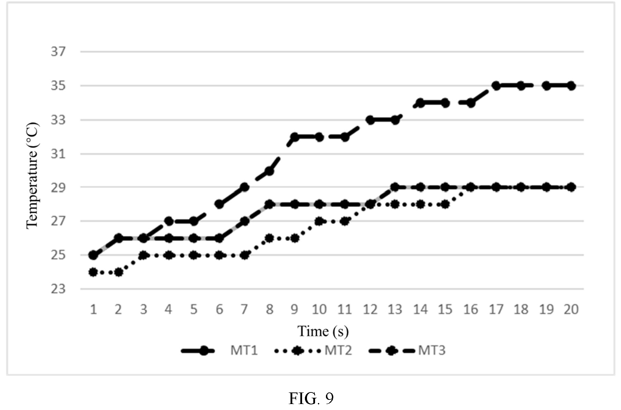

Referring to FIG. 4 and FIG. 9, FIG. 9 is a temperature recovery curve of temperature measured by the temperature measuring assembly 300 after ineffective occlusion by the cryoablation catheter 10 shown in FIG. 2. When the expandable assembly 200 does not completely occlude the vein ostium 510, a certain gap exists between the vein ostium 510 and the expandable assembly 200, and blood in the vein 50 flows to the atrium through the gap. After the expandable assembly 200 is inflated to the expandable configuration, a certain amount of mild liquid is injected into the main tube 120 through the first delivery passage. The mild liquid flows through the distal end of the first delivery passage into the blood in the vein 50. After the mild liquid is gradually mixed with the blood, part of the mixture flows into the atrium through the gap between the expandable assembly 200 and the vein ostium 510. Under the flushing of the blood flow, the temperature at the vein ostium 510 would be recovered at a relatively fast rate after dropping to the lowest point, with a relatively large recovery amplitude within a certain period of time. The expandable assembly 200 does not completely occlude the vein ostium 510. As shown in FIG. 4 and FIG. 9, the temperature detected by the first temperature measuring member MT1 is recovered rapidly, while the temperature recovery rates at the second temperature measuring member MT2 and the third temperature measuring member MT3 are relatively gentle. According to the temperature curves shown in FIG. 9, it can be determined that leakage occurs at the first temperature measuring member MT1. Under the function of the marker point 330 of the first temperature measuring member MT1, the operator can ensure complete occlusion of the vein ostium 510 by adjusting the orientation of the expandable assembly 200 according to the position of the first temperature measuring member MT1 in the vein 50.

In the cryoablation catheter 10 of the present application, the gas is delivered through the gas flow passage 130 of the main tube 120 to achieve expansion of the expandable assembly 200, so that the expandable assembly 200 is placed in the expanded configuration and occludes the vein ostium 510. During cryoablation, the cryoablation operation can be achieved through the cooperation of the gas flow passage, the second delivery passage, and the expandable assembly 200. Moreover, before ablation, the cryoablation catheter 10 delivers a harmless mild liquid into the vein 50 through the first delivery passage, and the temperature measuring assembly 300 on the expandable assembly 200 can be used to measure temperature changes to determine the occlusion state at this time, so as to determine whether there is a leakage between the expandable assembly 200 and the vein ostium 510. The usage amount of contrast agents and X-rays can also be reduced to avoid harm to the health of the physician and the patient. Moreover, the specific location of the leakage can be identified through the temperature measuring assembly 300, thereby helping the operator to quickly rotate and/or move the catheter, so as to adjust the direction and position of the expandable assembly 200 for effective occlusion, facilitating subsequent ablation treatment and ensuring therapeutic effect.

Referring to FIG. 1 to FIG. 5, the present application further provides a control method for a cryoablation catheter, which can be applied to the cryoablation catheter 10 according to any one of the above embodiments. The control method includes the following steps:
inserting a distal end of the cryoablation catheter 10 into a vein ostium 510;
delivering gas to an expandable assembly 200 to expand the expandable assembly 200 so as to occlude the vein ostium 510;
injecting a mild liquid into the vein 50 to mix with blood in the vein 50; and
acquiring a temperature at the vein ostium 510 by a temperature measuring assembly 300 and feeding back the temperature to a controller of a refrigeration device 60, and determining whether the expandable assembly 200 effectively occludes the vein ostium 510 by the controller based on a rate of temperature recovery at the vein ostium 510.

Referring to FIG. 1 to FIG. 5, when the cryoablation catheter 10 is used, the distal end of the cryoablation catheter 10 is inserted into the blood vessel, and the elongated shaft 100 is advanced so that the expandable assembly 200 moves to the vein ostium 510. Then, a gas is delivered from the gas flow passage 130 into the expandable assembly 200, so that the expandable assembly 200 is switched from the contracted configuration to the expanded configuration. In this case, the expandable assembly 200 is capable of occluding the vein ostium 510. After the expandable assembly 200 occludes the vein ostium 510, it is necessary to determine the effectiveness of occlusion of the vein ostium 510. The mild liquid is delivered from the first delivery passage into the vein 50. The temperature of blood at the vein ostium 510 is measured by the temperature measuring assembly 300 and fed the temperature back to the controller to determine whether the expandable assembly 200 effectively occludes the vein ostium 510.

For determining whether effective occlusion is achieved, when it is determined by the controller that the temperature detected by the temperature measuring assembly 300 is recovered stably, it indicates that the expandable assembly 200 can effectively occlude the vein ostium 510. When it is determined by the controller that that the temperature detected by the temperature measuring assembly 300 is recovered rapidly, it indicates that the expandable assembly 200 does not effectively occlude the vein ostium 510. In this case, there is a leakage in at least one location of the expandable assembly 200, and the orientation of the cryoablation catheter 10 needs to be adjusted until the expandable assembly 200 effectively occludes the vein ostium 510. Meanwhile, the mild liquid does not cause damage to the blood vessels, and can effectively avoid the situation where patients are intolerant to contrast agents, reduce X-ray irradiation, and avoid damage to the patient and the operator, as shown in FIG. 4 and FIG. 5. After complete occlusion, cryoablation can be performed with the cryoablation catheter 10 on the lesion site near the vein ostium 510, so as to achieve the therapeutic purpose.

In an embodiment, determining whether the expandable assembly 200 effectively occludes the vein ostium 510 by the controller based on a temperature change at the vein ostium 510 includes the following steps:
injecting the mild liquid into the vein 50, and acquiring a first temperature at the vein ostium 510 by the temperature measuring assembly 300 after mixing of the mild liquid and the blood; and
after a preset time period, acquiring a second temperature at the vein ostium 510 by the temperature measuring assembly 300 and feeding back the second temperature to the controller, the second temperature being greater than the first temperature, and determining whether the expandable assembly 200 effectively occludes the vein ostium 510 by the controller based on a rate of recovery from the first temperature to the second temperature.

Blood in the vein 50 has an initial temperature. After the expandable assembly 200 occludes the vein ostium 510, the initial temperature at the vein ostium 510 is acquired by the temperature measuring assembly 300 and fed back to the controller. After the mild liquid is introduced into the vein 50, the mild liquid is mixed with the blood, and it is detected by the temperature measuring assembly 300 that the temperature of the blood in the vein 50 drops to a first temperature, which is lower than the initial temperature. The first temperature is the lowest temperature after mixing of the mild liquid and the blood. After a preset time period, it is detected by the temperature measuring assembly 300 that the temperature of the blood in the vein 50 rises, and this temperature is recorded as a second temperature, which is higher than the first temperature. At this time, the controller can determine whether effective occlusion is achieved according to the rate of recovery from the first temperature to the second temperature. When it is determined by the controller that that the second temperature is recovered stably, the expandable assembly 200 effectively occludes the vein ostium 510; when it is determined by the controller that that the second temperature is recovered rapidly, the expandable assembly 200 does not effectively occlude the vein ostium 510.

In an embodiment, determining whether the expandable assembly 200 effectively occludes the vein ostium 510 by the controller based on the rate of recovery of the second temperature further includes the following steps:
the controller determining that the expandable assembly 200 effectively occludes the vein ostium 510 when it is determined by the controller that the second temperature is recovered at a first recovery rate;
the controller determining that the expandable assembly 200 does not effectively occlude the vein ostium 510 when it is determined by the controller that the second temperature is recovered at a second recovery rate;
wherein the first recovery rate is less than the second recovery rate.

When it is determined by the controller that the second temperature is recovered at the first recovery rate, the second temperature is recovered stably, indicating that the expandable assembly 200 effectively occludes the vein ostium 510. When it is determined by the controller that the second temperature is recovered at the second recovery rate, the second temperature is recovered rapidly, indicating that the expandable assembly 200 does not effectively occlude the vein ostium 510.

In an embodiment, a plurality of temperature measuring assemblies 300 are provided, and the plurality of temperature measuring assemblies 300 are disposed on the expandable assembly 200 and distributed along a circumferential direction so as to be in contact with an inner wall of the vein ostium 510, and the control method further includes the following steps:

Acquiring, by the temperature measuring assemblies 300, temperatures at positions of the vein ostium 510 corresponding to the temperature measuring assemblies 300;
after injecting the mild liquid, determining that the expandable assembly 200 effectively occludes the vein ostium 510, when the temperatures acquired by each of the temperature measuring assemblies 300 are recovered stably;
or when the temperatures acquired by at least one of the temperature measuring assemblies 300 are recovered rapidly, determining that the expandable assembly 200 has a leakage at a location corresponding to that temperature measuring assembly 300;
rotating the cryoablation catheter 10 until the temperatures acquired by each of the temperature measuring assemblies 300 are recovered stably.

After the expandable assembly 200 occludes the vein ostium 510, the plurality of temperature measuring assemblies 300 are distributed at different positions of the vein ostium 510. After the mild liquid is introduced, each temperature measuring assembly 300 can respectively acquire a temperature at a corresponding position of the vein ostium 510. When the temperatures acquired by each of the temperature measuring assemblies 300 are recovered stably, the controller determines that the expandable assembly 200 effectively occludes the vein ostium 510. When the temperature acquired by at least one temperature measuring assembly 300 is recovered rapidly, it indicates that a gap exists between the expandable assembly 200 and the vein ostium 510 at the position corresponding to the temperature measuring assembly 300, and effective occlusion is not achieved. In this case, it is necessary to adjust the direction and position of the expandable assembly 200 until the temperatures acquired by each of the temperature measuring assemblies 300 are recovered stably, so as to achieve effective occlusion of the vein ostium 510.

Referring to FIG. 1, the present application further provides a cryoablation system, including a refrigeration device 60, a display control device 70, and the cryoablation catheter 10 of any one of the above technical features. The display control device 70 is connected to the refrigeration device 60. A control handle 400 of the cryoablation catheter 10 has a proximal end connected to the refrigeration device 60, and a distal end inserted into a vein ostium 510 for occluding the vein ostium 510. By employing the cryoablation catheter 10 of the above embodiments in the cryoablation system of the present application, the cryoablation catheter 10 determines whether effective occlusion is achieved by delivering a mild liquid into the vein 50 in combination with temperature measurement, reduces the usage amount of contrast agent and X-rays, and facilitates determination of the specific location of the leakage when leakage occurs, so as to perform effective occlusion and ensure therapeutic effect. The refrigeration device 60 has a controller therein, and the display control device 70 has a display screen therein. The controller is electrically connected to the display screen and the temperature measuring assembly 300, so as to display the temperature transmitted by the temperature measuring assembly 300 and display the occlusion state on the display screen.

The above-described technical features may be arbitrarily combined. To make the description concise, not all possible combinations of the technical features in the above-described embodiments are described. However, as long as there is no contradiction in the combinations of these technical features, the combinations should be considered to be within the scope of this specification.

The embodiments above only represent several implementation modes of the present application, and the description thereof is relatively specific and detailed, but it should not be construed as limiting the scope of the patent. It should be noted that for those ordinary skilled in the art, various modifications and improvements may be made without departing from the concept of the present application, and all these modifications and improvements are within the protection scope of the present application. Therefore, the scope of protection of the patent application should be subject to the appended claims. The specification and the accompanying drawings may be used to interpret the content of the claims.

## Claims

1. A cryoablation catheter, comprising an elongated shaft, an expandable assembly, and a temperature measuring assembly, wherein the expandable assembly is disposed at a distal end of the elongated shaft and has an expanded configuration and a contracted configuration, and the expandable assembly, when in the expanded configuration, is capable of occluding a vein ostium;
the elongated shaft comprises an outer tube and a main tube, the outer tube is sleeved over the main tube and connected at a distal end thereof to the expandable assembly, the outer tube and the main tube define a gas flow passage therebetween, the gas flow passage is in communication with the expandable assembly, the main tube has a first delivery passage extending in an axial direction, the first delivery passage extends through the main tube and is configured to deliver a mild liquid into the vein; and
the temperature measuring assembly is disposed on the expandable assembly and is capable of measuring a temperature at the vein ostium to determine effectiveness of occlusion by the expandable assembly.

2. The cryoablation catheter according to claim 1, wherein the expandable assembly comprises a first expandable element and a second expandable element, the first expandable element envelops the second expandable element, an inner cavity of the second expandable element is in communication with the gas flow passage, and the temperature measuring assembly is disposed between the first expandable element and the second expandable element and disposed on an outer wall of the second expandable element.

3. The cryoablation catheter according to claim 1, wherein a plurality of temperature measuring assemblies are provided, and the plurality of temperature measuring assemblies are disposed on the expandable assembly;
a spacing is provided between adjacent temperature measuring assemblies, or the plurality of temperature measuring assemblies are distributed all over the expandable assembly.

4. The cryoablation catheter according to any one of claims 1 to 3, wherein the temperature measuring assembly comprises a temperature measuring element and a fixing component, the fixing component fixes the temperature measuring element to the expandable assembly, and the temperature measuring element and the fixing component are stretched along with expansion of the expandable assembly.

5. The cryoablation catheter according to claim 4, wherein the temperature measuring element comprises a linear element and a temperature measuring point, the linear element is disposed on the fixing component, and the temperature measuring point is disposed at a distal end or an intermediate region of the linear element.

6. The cryoablation catheter according to claim 4, wherein the cryoablation catheter further comprises a marker point, the marker point is disposed on the fixing component, and the marker point is configured to mark a relative position of the temperature measuring element with respect to the vein ostium.

7. The cryoablation catheter according to claim 6, wherein each of the fixing components has at least one marker point, and the marker points on the respective fixing components are different from one another.

8. The cryoablation catheter according to claim 6, wherein at least part of the fixing components have at least one marker point, and among the fixing components having the marker points, the marker points on the respective fixing components are different from one another.

9. The cryoablation catheter according to claim 8, wherein the fixing components having the marker points are adjacent to each other, and at least one fixing component having the marker points is disposed between adjacent fixing components having no marker points, and at least one fixing component having no marker points is disposed between adjacent fixing components having the marker points.

10. The cryoablation catheter according to claim 6, wherein among the fixing components having the marker points, at least one of a number, shape, or length of the marker points is different.

11. A control method for a cryoablation catheter, applied to the cryoablation catheter according to any one of claims 1 to 10, the control method comprising the following steps:
inserting a distal end of the cryoablation catheter into a vein ostium;
delivering a gas to an expandable assembly to expand the expandable assembly so as to occlude the vein ostium;
injecting a mild liquid into the vein to mix with blood in the vein; and
acquiring a temperature at the vein ostium by a temperature measuring assembly and feeding back the temperature to a controller of a refrigeration device, and determining whether the expandable assembly effectively occludes the vein ostium by the controller based on a rate of temperature recovery at the vein ostium.

12. The control method according to claim 11, wherein determining whether the expandable assembly effectively occludes the vein ostium by the controllerbased on the temperature change at the vein ostium comprises the following steps:
injecting the mild liquid into the vein, and acquiring a first temperature at the vein ostium by the temperature measuring assembly after mixing of the mild liquid with the blood; and
after a preset time period, acquiring a second temperature at the vein ostium by the temperature measuring assembly and feeding back the second temperature to the controller, wherein the second temperature is greater than the first temperature, and determining whether the expandable assembly effectively occludes the vein ostium by the controllerbased on a rate of recovery from the first temperature to the second temperature.

13. The control method according to claim 12, wherein determining whether the expandable assembly effectively occludes the vein ostium by the controllerbased on the rate of recovery to the second temperature further comprises the following steps:
the controller determining that the expandable assembly effectively occludes the vein ostium when it is determined that the second temperature is recovered at a first recovery rate by the controller;
the controller determining that the expandable assembly does not effectively occlude the vein ostium when it is determined that the second temperature is recovered at a second recovery rate by the controller;
wherein the first recovery rate is less than the second recovery rate.

14. The control method according to claim 11, wherein a plurality of temperature measuring assemblies are provided, and the plurality of temperature measuring assemblies are disposed on the expandable assembly and distributed along a circumferential direction so as to be in contact with an inner wall of the vein ostium, and the control method further comprises the following steps:
injecting the mild liquid, and acquiring, by the temperature measuring assemblies, temperatures at positions of the vein ostium corresponding to the temperature measuring assemblies;
determining that the expandable assembly effectively occludes the vein ostium, when the temperatures acquired by each of the temperature measuring assemblies are recovered stably;
or when the temperatures acquired by at least one of the temperature measuring assemblies are recovered rapidly, determining that the expandable assembly has a leakage at a location corresponding to that temperature measuring assembly;
rotating the cryoablation catheter until the temperatures acquired by each of the temperature measuring assemblies are recovered stably.

15. A cryoablation system, comprising a refrigeration device, a display control device, and the cryoablation catheter according to any one of claims 1 to 10, wherein the display control device is connected to the refrigeration device, and a control handle of the cryoablation catheter has a proximal end connected to the refrigeration device and a distal end inserted into a vein ostium for occluding the vein ostium.
